# EUROPEAN PATENT APPLICATION

(11) **EP 1 609 788 A1**
(43) Date of publication of application: **28.12.2005**
(21) Application number: 04725182.2
(22) Date of filing: 01.04.2004
(51) Int. Cl.: C07D 401/06, A61K 31/4725, A61P 9/10, A61P 43/00

(54) **NOVEL CRYSTAL OF FLUOROBENZAMIDE DERIVATIVE**

(30) Priority: 02.04.2003 JP 2003099411
(71) Applicant: Astellas Pharma Inc., Tokyo 103-8411 (JP)
(72) Inventor: YOSHIDA, Shinya, Astellas Pharma Inc., Chuo-ku, Tokyo 103-8411 (JP); MARUYAMA, Kiyotaka, Astellas Pharma Inc., Chuo-ku, Tokyo 103-8411 (JP); WATANABE, Toshihiro, Astellas Pharma Inc., Chuo-ku, Tokyo 103-8411 (JP); YAMAGUCHI, Sou, Astellas Pharma Inc., Chuo-ku, Tokyo 103-8411 (JP)
(74) Representative: HOFFMANN EITLE
(86) International application number: PCT/JP2004/004794
(87) International publication number: WO 2004/089933

(57) **Abstract**

There are provided novel crystals of (-)-N-{2-[(R)-3-(6,7-dimethoxy-1,2,3,4-tetrahydroisoquinoline-2-carbonyl)piperidino]ethyl}-4-fluorobenzamide monophosphate.

## Description

### Technical Field

The present invention relates to novel crystals of (-)-*N*-{2-[(*R*)-3-(6,7-dimethoxy-1,2,3,4-tetrahydroisoquinoline-2-carbonyl)piperidino]ethyl}-4-fluorobenzamide monophosphate (hereinafter, referred to as "compound A") and to a process for preparing the same.

### Background Art

The compound A has been known to have an inhibitory action for I_{f} current and shows a strong and specific activity selectively lowering a heart beat and decreasing oxygen consumption of heart muscle whereby it is useful as a preventive and/or treating agent for diseases of circulatory system such as ischemic heart diseases (e.g., angina pectoris and myocardial infarction), congestive heart failure, arrhythmia, etc. (Patent Document 1).

In Example 49 of the document, the compound A is specifically disclosed and is mentioned that its crystal has a melting point of 197 to 199°C. However, there is neither disclosure nor suggestion that the compound A has crystal polymorphism.

In the meanwhile, in a compound where crystal polymorphism is present, various properties thereof differ depending upon crystal form and, therefore, even the same compound may sometimes show entirely different function and effect. Particularly in pharmaceuticals, when a compound having different function and effect is used with a presumption that it has the same efficacy, there is a possibility that function and effect which are different from expected ones are noted whereby unexpected accident is resulted. Accordingly, there is a demand to provide a compound of the same quality which is able to expect predetermined function and effect at all times. Therefore, when a compound in which crystal polymorphism is present is used as a pharmaceutical, it is necessary that a certain single crystal of the compound is always provided in order to ensure the uniform quality and certain function and effect which are demanded for pharmaceuticals.

Under such circumstances, in view of providing the active ingredient for pharmaceuticals, there has been a brisk demand for crystals of the compound A by which uniform quality and certain function and effect are able to be ensured and a stable supply in an industrial production is possible and for a process for preparing the same.
[Patent Document 1] WO 00/75133

### Disclosure of the Invention

The present inventors have carried out intensive studies for providing the crystals of the compound A and have unexpectedly found that, due to the difference in precise preparation condition, the resulting crystal varies or, in other words, crystal polymorphism is present in the compound A.

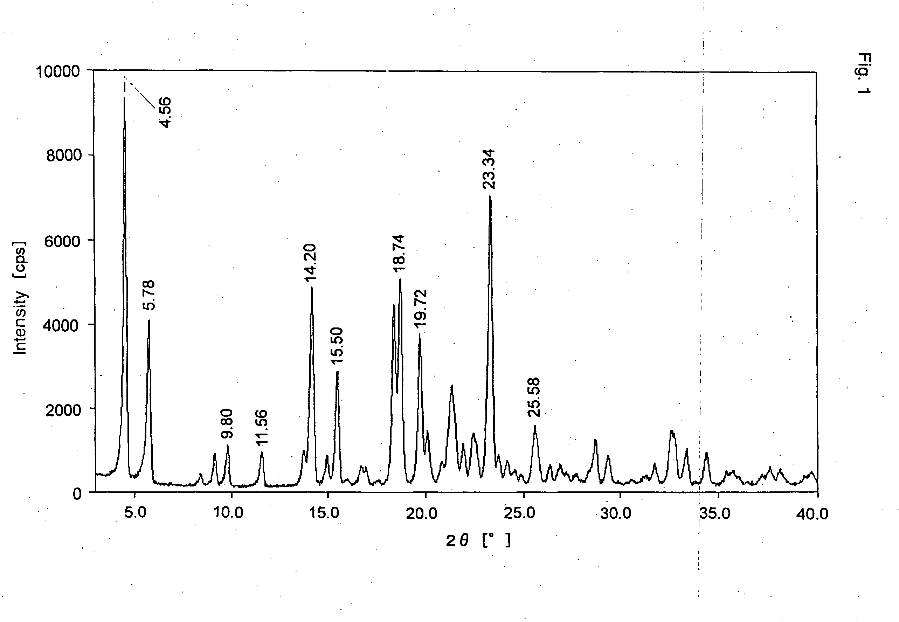

It has been also found that, among the above, crystal having a X-ray powder diffraction pattern shown in Fig. 1 (hereinafter, the crystal will be referred to as "α-type crystal") has particularly good hygroscopicity and, therefore, it is advantageous in view of handling of the active ingredient and of making into pharmaceutical preparations and further that it is excellent in terms of stable supply in view of industrial production.

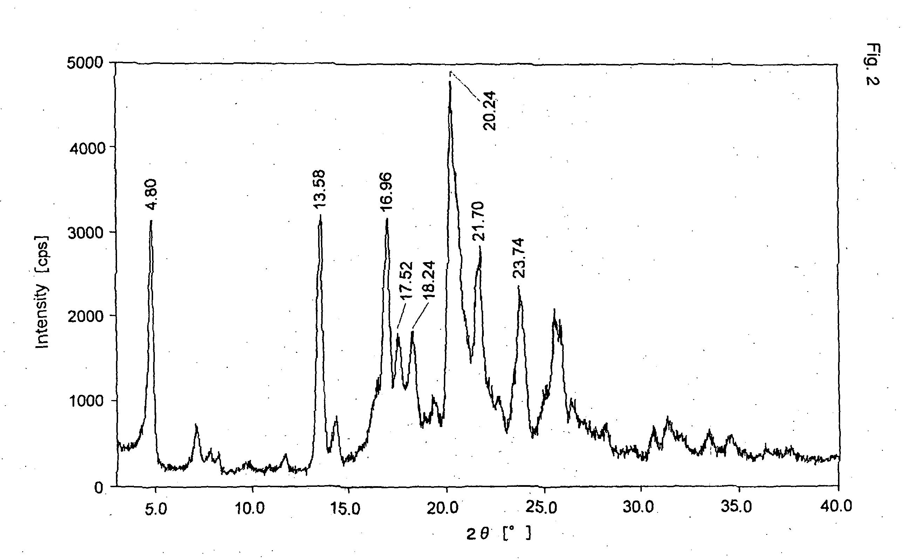

The presence of crystal having a X-ray powder diffraction pattern shown in Fig. 2 which is different from the α-type crystal of the compound A (hereinafter, the crystal will be referred to as "β-type crystal") has been found as well.

Thus, the present invention has been achieved on the basis of such findings and, in accordance with the present invention, there are provided crystal of the compound A which is characterized in having main peaks near 19.36, 6.23, 4.73 and 3.81 Å of lattice spacing in a X-ray powder diffraction pattern obtained by the use of Cu-Kα radiation; crystal of the compound A which is characterized in that lattice spacing and relative intensity are preferably those as shown in Table 1 in a X-ray powder diffraction pattern obtained by the use of Cu-Kα radiation; and crystal of the compound A which is characterized in that, in a DSC curve, the temperature of endothermic peak is near 206 to 207°C.

**(Table 1)**

| Lattice Spacing (Å) | Relative Intensity |
|---|---|
| 19.36 | strong |
| 15.28 | slightly strong |
| 6.23 | strong |
| 5.71 | slightly strong |
| 4.73 | strong |
| 4.50 | slightly strong |
| 3.81 | strong |

Further, in accordance with the present invention, there are provided crystal of the compound A which is characterized in having main peaks near 18.40, 6.52, 5.22, 4.38 and 4.09 Å of lattice spacing in a X-ray powder diffraction pattern obtained by the use of Cu-Kα radiation; crystal of the compound A which is characterized in that lattice spacing and relative intensity are preferably those as shown in Table 2 in a X-ray powder diffraction pattern obtained by the use of Cu-Kα radiation; and crystal of the compound A which is characterized in that, in a DSC curve, the temperature of endothermic peak is near 176 to 177°C.

**(Table 2)**

| Lattice Spacing (Å) | Relative Intensity |
|---|---|
| 18.40 | strong |
| 6.52 | strong |
| 5.22 | strong |
| 5.06 | slightly strong |
| 4.86 | slightly strong |
| 4.38 | strong |
| 4.09 | strong |
| 3.74 | slightly strong |

Incidentally, in the recognition of identity of crystal, crystal lattice spacing and pattern in general are important in X-ray powder diffraction pattern in view of the data and relative intensity is more or less variable depending upon direction of crystal growth, size of particle and measuring condition whereby a strict understanding therefor should not be done. Further, although the present invention relates to pure α-type crystal and pure β-type crystal of compound A, a mixture which is able to be understood to be substantially identical with those α-type crystal and β-type crystal is also covered by the present invention.

The present invention will now be illustrated in detail as hereunder.

In the data obtained from various kinds of spectra, there may be some errors depending upon direction of crystal growth, size of particle and measuring condition. Therefore, in the present specification, the term "near" used in the value of lattice spacing in the X-ray powder diffraction pattern means an approximate lattice spacing value, preferably means that it may be within 1.0 Å before and after the value and, more preferably, means that it may be within 0.3 Å before and after the value. In addition, the term "near" used in the value for the temperature of endothermic peak in a DSC curve means an approximate endothermic peak value, preferably means that it may be within 2°C before and after the value and, more preferably, means that it may be within 1 °C before and after the value.

The α-type crystal of the compound A of the present invention is able to be manufactured in such a manner that, when the corresponding free substance is made into a salt using phosphoric acid, it is carried out at the temperature of about 30°C in an ethanol (EtOH) solvent or in an EtOH solvent to which a few amount of water is added.

The β-type crystal of the compound A of the present invention is able to be manufactured in such a manner that, when the corresponding free substance is made into a salt using phosphoric acid, it is carried out by cooling at about 5°C in an EtOH solvent or in an EtOH solvent to which a few amount of water is added.

The α-type crystal of the compound A of the present invention is also able to be manufactured in such a manner that the resulting crystal is recrystallized in an EtOH solvent or in an EtOH solvent to which water is added. In conducting the recrystallization, it is possible to conduct by inoculation of seed crystal and, in order to prevent a rapid progress of crystallization, the temperature for inoculation is preferably room temperature or higher, more preferably 30°C or higher or, particularly preferably, 50°C or higher.

The α-type crystal of the compound A of the present invention is also able to be manufactured in such a manner that a mixture of α-type crystal and β-type crystal or β-type crystal is suspended in an EtOH solvent or in an EtOH solvent to which water is added and stirred at about 40°C.

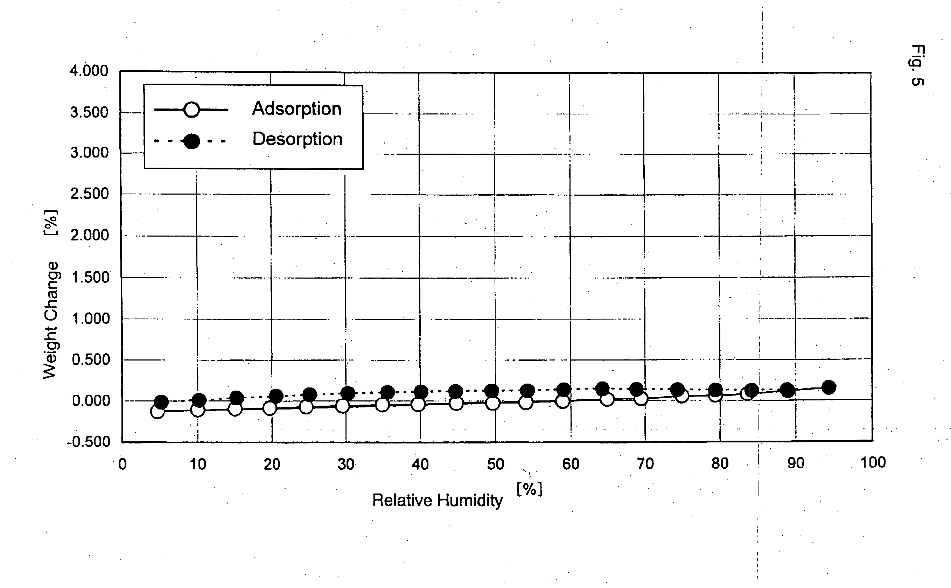

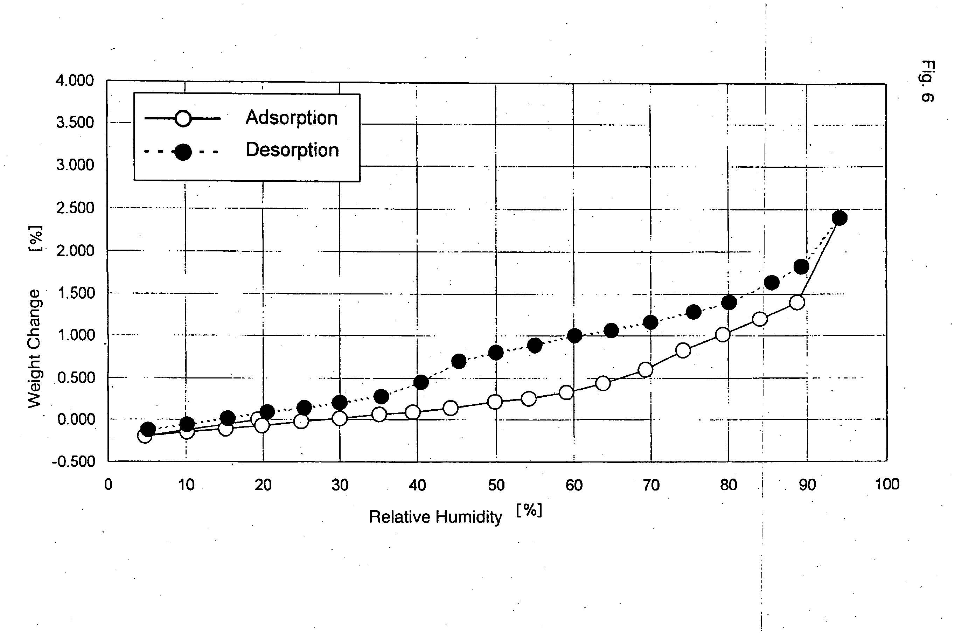

Hygroscopicity of the two kinds of novel crystals of the compound A according to the present invention are shown in Fig. 5 and Fig. 6.

As shown in Fig. 5 and Fig. 6, the α-type crystal of the compound A of the present invention is superior to the β-type crystal in terms of hygroscopicity and, therefore, it is advantageous in view of handling of active ingredient and of manufacture of pharmaceutical preparations. When the α-type crystal of the compound A is manufactured as a single crystal form by the above-mentioned manufacturing method, it is possible to ensure a certain quality demanded as pharmaceuticals. Incidentally, the α-type crystal and the β-type crystal of the compound A of the present invention'are morphologically pure.

### Best Mode for Carrying Out the Invention

The present invention will now be illustrated in more detail by way of the following Referential Examples and Examples, although the present invention is not limited to those Examples.

Incidentally, TGA-DSC thermal analysis was conducted as follows.

### <TGA-DSC thermal analysis>

A sample (5 mg) was filled in a sample container, a heating furnace was heated according to a certain heating rate (10°C/minute) and changes in calorie (DSC) and changes in thermogravity (TGA) were continuously measured and recorded. Incidentally, handling of the apparatus including data processing was conducted in accordance with the methods and procedures instructed by each of the apparatuses (Instrument used: DSC 2910 and Hi-Res TGA 2950).

Purity of the compound was measured using a high-performance liquid chromatography (hereinafter, referred to as "HPLC").

With regard to NMR, (CH₃)₄Si was used as an internal standard and, unless otherwise mentioned, it is shown by a peak value (ppm) in ¹H-NMR using DMSO (dimethyl sulfoxide) -*d*₆ as a solvent for the measurement. Referential Example 1-a

To 120.0 g of 2-(4-fluorophenyl)-4,5-dihydrooxazole were added 1,200 ml of toluene and 137.07 g of ethyl (R)-piperidine-3-carboxylate. Ethyl (R)-piperidine-3-carboxylate was washed with 600 ml of toluene. After that, 145.1 g of p-toluenesulfonic acid monohydrate was added thereto followed by washing with 600 ml of toluene. Then the reaction mixture was heated and the solvent was distilled at ordinary pressure to evaporate 600 ml thereof. After that, the reaction mixture was stirred for 27 hours under reflux. After the reaction mixture was cooled, 960 ml of ethyl acetate (EtOAc) and 960 ml of 4% (w/v) aqueous solution of NaHCO₃ were added. The reaction mixture was allowed to stand and separation was conducted at about 35°C. The resulted organic layer was washed twice with 960 ml of a 4% (w/v) aqueous solution of NaHCO₃. The organic layer was concentrated *in vacuo* to give ethyl (R)-1-{2-[(4-fluorobenzoyl)amino]ethyl}piperidine-3- carboxylate having a purity of 82.8%.
NMR: δ 1.16 (3H, t), 1.35-1.80 (4H, m), 2.05-2.10 (1H, m), 2.20-2.26 (1 H, m), 2.45-2.51 (3H, m), 2.65-2.70 (1 H, m), 2.85-2.90 (1 H, m), 3.30-3.38 (2H, m), 4.05 (2H, q), 7.25-7.33 (2H, m), 7.85-7.95 (2H, m), 8.35-8.40 (1 H, m).
FAB-MS m/z: 323 (M⁺ + 1).

### Referential Example 1-b

To 230 g of the compound of Referential Example 1-a was added 690 ml of EtOH and then 345 ml of water was added thereto. After cooling, an aqueous solution of NaOH (42.8 g NaOH/480 ml water) was added followed by stirring for 2 hours at 25°C or a lower temperature. After cooling, concentrated hydrochloric acid was added to the reaction solution to adjust to pH 3.0. This solution was concentrated *in vacuo*, 1,000 ml of toluene was added to the residue and the mixture was concentrated *in vacuo* to give (R)-1-{2-[(4-fluorobenzoyl)amino]ethyl}piperidine-3- carboxylic acid having a purity of 86.3%.
NMR (90°C): δ 1.46-1.60 (1 H, m), 1.79-2.05 (3H, m), 2.75-3.60 (7H, m), 3.68 (2H, q), 7.20-7.27 (2H, m), 7.95-8.03 (2H, m), 8.74 (1 H, brs).
FAB-MS m/z: 295 (M⁺ + 1).

### Referential Example 1-c

To 206.4 g of the compound of Referential Example 1-b were added 810 ml of *N,N*-dimethylformamide (DMF) and 120.8 g of 6,7-dimethoxy-1,2,3,4-tetrahydroisoquinoline monohydrochloride followed by stirring and cooling. After that 53.22 g of triethylamine was added at 12°C or a lower temperature and then 217 ml of DMF was added. Then 21.32 g of 1 H-1,2,3-benzotriazol-1-ol (HOBt) was added at 5°C or a lower temperature and then 121.0 g of 1-ethyl-3-(3-dimethylaminopropyl)carbodiimide hydrochloride (WSC.HCl) was added at 5°C or a lower temperature: The reaction mixture was stirred at 0 to 4°C for 15.5 hours. To the reaction mixture were added 340 ml of water, 2,000 ml of EtOAc and 550 ml of a 8% (w/v) aqueous solution of NaOH followed by separating. EtOAc (1,000 ml) was added to the aqueous layer followed by separating. After that, the organic layers were mixed and washed for two times with 700 ml of a 8% (w/v) aqueous solution of NaOH and 300 ml of water. After the organic layer was washed with 900 ml of water, it was concentrated *in vacuo* to give (-)-N-{2-[(R)-3-(6,7-dimethoxy-1,2,3,4-tetrahydroisoquinoline-2-carbonyl)piperidino]ethyl}-4-fluorobenzamide having a purity of 83.9%.
NMR (CDCl₃): δ 1.54-1.68 (2H, m), 1.75-1.87 (2H, m), 2.05-2.95 (9H, m), 3.45-3.63 (2H, m), 3.70 (1 H, t), 3.75-3.82 (1 H, m), 3.84 (3H, s), 3.85 (3H, s), 4.59 (1 H, br), 4.62 (1 H, br), 6.55-6.65 (2H, m), 6.95 (1 H, br), 7.11 (2H, t), 7.77-7.88 (2H, m).
FAB-MS m/z: 470 (M⁺ + 1).

### Example 1

### (Manufacture of α-type crystal of compound A)

EtOH was added to the compound (11.94 g) prepared by the same manner as in Referential Example 1 to dissolve so that the total amount was made 97.8 g. To 32.6 g of this solution were added 5 ml of EtOH and 0.47 g of water, then 0.86 g of 85% phosphoric acid was added thereto followed by washing with 5 ml of EtOH. The crystallizing solution was stirred at 30°C for one night. Crystals were filtered, washed with EtOH and dried *in vacuo* to give 3.38 g of (-)-N-{2-[(R)-3-(6,7-dimethoxy-1,2,3,4-tetrahydroisoquinoline-2-carbonyl)piperidino]ethyl}-4-fluorobenzamide monophosphate (α-type crystal) having a purity of 95.8%.
NMR: δ 1.30-1.47 (1H, m), 1.60-1.85 (3H, m), 2.30-2.85 (6H, m), 3.00-3.20 (3H, m), 3.40-3.56 (2H, m), 3.60-3.78 (8H, m), 4.50 (1 H, q), 4.64 (1 H, q), 6.73 (1 H, s), 6.78, 6.86 (1 H, s in combination), 7.23-7.33 (2H, m), 7.90-8.00 (2H, m), 8.65-8.77 (1H, m).
FAB-MS m/z: 470 (M⁺ + 1).

### Example 2

### (Manufacture of β-type crystal of compound A)

EtOH was added to the compound (11.94 g) prepared by the same manner as in Referential Example 1 to dissolve so that the total amount was made 97.8 g. To 32.6 g of this solution were added 5 ml of EtOH and 0.47 g of water, then 0.86 g of 85% phosphoric acid was added thereto followed by washing with 5 ml of EtOH. The crystallizing solution was stirred at 5°C for one night. Crystals were filtered, washed with EtOH and dried *in vacuo* to give 3.27 g of (-)-N-{2-[(R)-3-(6,7-dimethoxy-1,2,3,4-tetrahydroisoquinoline-2-carbonyl)piperidino]ethyl}-4-fluorobenzamide monophosphate (β-type crystal) having a purity of 96.4%.
NMR: δ 1.30-1.48 (1H, m), 1.60-1.85 (3H, m), 2.26-2.85 (6H, m), 3.00-3.20 (3H, m), 3.40-3.55 (2H, m), 3.60-3.78 (8H, m), 4.50 (1 H, q), 4.63 (1 H, q), 6.73 (1 H, s), 6.78, 6.86 (1 H, s in combination), 7.22-7.33 (2H, m), 7.88-8.00 (2H, m), 8.64-8.77 (1H, m).
FAB-MS m/z: 470 (M⁺ + 1).
Melting point: 195.5-196.3°C

A chart showing the X-ray powder diffraction data of the compound of Example 2 is shown in Fig. 2, a chart showing a TGA-DSC thermal analysis data is shown in Fig. 4 and a chart showing a hygroscopicity is shown in Fig. 6.

### Referential Example 2-a

To a solution comprising 110 liters of water and 36.8 kg of potassium carbonate was added 27.3 kg of 2-bromoethylamine monohydrobromide at -5°C or a lower temperature with stirring. To the reaction mixture was added 100 liters of EtOAc and then 21.1 kg of 4-fluorobenzoyl chloride was added thereto at 5°C or a lower temperature with stirring. To this was added 10 liters of EtOAc. The reaction mixture was monitored by HPLC measurement to confirm the production of N-(2-bromoethyl)-4-fluorobenzamide having a purity of 85.2%.

### Referential Example 2-b

The reaction mixture of Referential Example 2-a was heated and stirred at 45 to 52°C for 4 hours. To the reaction mixture was added 40 liters of toluene and the mixture was allowed to stand to separate at about 35°C. The organic layer was washed with 80 liters of water. As a result, a solution of 2-(4-fluorophenyl)-4,5- dihydrooxazole having a purity of 98% in toluene-EtOAc was prepared.

### Referential Example 2-c

To the toluene-EtOAc solution prepared in Referential Example 2-b were added 440 liters of toluene, 25.1 kg of ethyl (R)-piperidine-3-carboxylate and 26.6 kg of p-toluenesulfonic acid monohydrate, the mixture was heated and the solvent was distilled at ordinary pressure to evaporate 260 liters. After that, stirring was conducted with refluxing for 32 hours. After the reaction mixture was cooled, 260 liters of EtOAc and 180 liters of a 4% (w/v) aqueous solution of NaHCO₃ were added. The reaction mixture was allowed to stand and separation was conducted at about 30°C. The organic layer was washed with 180 liters of a 4% (w/v) aqueous solution of NaHCO₃ twice. The organic layer was concentrated *in vacuo* to give ethyl (R)-1-{2-[(4-fluorobenzoyl)amino]ethyl}piperidine-3-carboxylate having a purity of 86.7%.

### Referential Example 2-d

To the compound prepared in Referential Example 2-c was added 260 liters of EtOH and then 64 liters of water was added thereto. After cooling, an aqueous solution of NaOH (8.0 kg NaOH/90 liters water) was added thereto followed by stirring at 25°C or a lower temperature for 2 hours. After cooling, concentrated hydrochloric acid was added to the reaction to adjust pH to 3.06. This solution was concentrated *in vacuo*, 190 liters of toluene was added to the residue and the mixture was concentrated *in vacuo.* To the residue was added 190 liters of toluene followed by concentrating *in vacuo.* To the residue was added 190 liters of toluene followed by concentrating *in vacuo* to give (R)-1-{2-[(4-fluorobenzoyl)amino]ethyl}piperidine-3- carboxylic acid having a purity of 90.4%.

### Referential Example 2-e

To the compound prepared in Referential Example 2-d were added 200 liters of DMF and 23.0 kg of 6,7-dimethoxy-1,2,3,4-tetrahydroisoquinoline monohydrochloride followed by stirring and cooling. Then 10.1 kg of triethylamine was added at 10°C or a lower temperature and 4.0 kg of HOBt was added thereto at 5°C or a lower temperature. After that, 23.0 kg of WSC.HCl was added at 5°C or a lower temperature followed by stirring at -4 to 4°C for one night. To the reaction mixture were added 64 liters of water, 380 liters of EtOAc and 105 liters of a 8% (w/v) aqueous solution of NaOH followed by separating. To the aqueous layer was added 190 liters of EtOAc followed by separating. After that, organic layers were combined and washed for two times with 130 liters of a 8% (w/v) aqueous solution of NaOH and 57 liters of water. After the organic layer was washed with 170 liters of water, it was concentrated *in vacuo* to give (-)-N-{2-[(R)-3-(6,7-dimethoxy-1,2,3,4-tetrahydroisoquinoline-2-carbonyl)piperidino]ethyl}-4-fluorobenzamide having a purity of 79%. EtOH (140 liters) was added thereto to dissolve.

### Example 3

### (Manufacture of α-type crystal of compound A)

To EtOH solution prepared in Referential Example 2-e was added 330 liters of EtOH. After that, 11.5 kg of 85% phosphoric acid and 52 liters of water were added thereto followed by heating and the resulting solution was filtered. The filtrate was heated with stirring and cooled and the compound having the same crystal form as Example 1 was added as seed crystals at about 55°C followed by cooling and stirring at about 0 to 5°C. The crystals separated out therefrom were filtered, washed with EtOH and dried *in vacuo* to give 36.51 kg of (-)-N-{2-[(R)-3-(6,7-dimethoxy-1,2,3,4-tetrahydroisoquinoline-2-carbonyl)piperidino]ethyl}-4-fluorobenzamide monophosphate (α-type crystal) having a purity of 98.9%.
NMR: δ 1.30-1.50 (1H, m), 1.60-1.85 (3H, m), 2.30-2.85 (6H, m), 3.05-3.20 (3H, m), 3.40-3.57 (2H, m), 3.60-3.77 (8H, m), 4.50 (1 H, q), 4.63 (1 H, q), 6.72 (1 H, s), 6.77, 6.86 (1 H, s in combination), 7.27 (2H, t), 7.88-8.00 (2H, m), 8.70 (1 H, br).
FAB-MS m/z: 470 (M⁺ + 1).
Melting point: 203.8-204.7°C

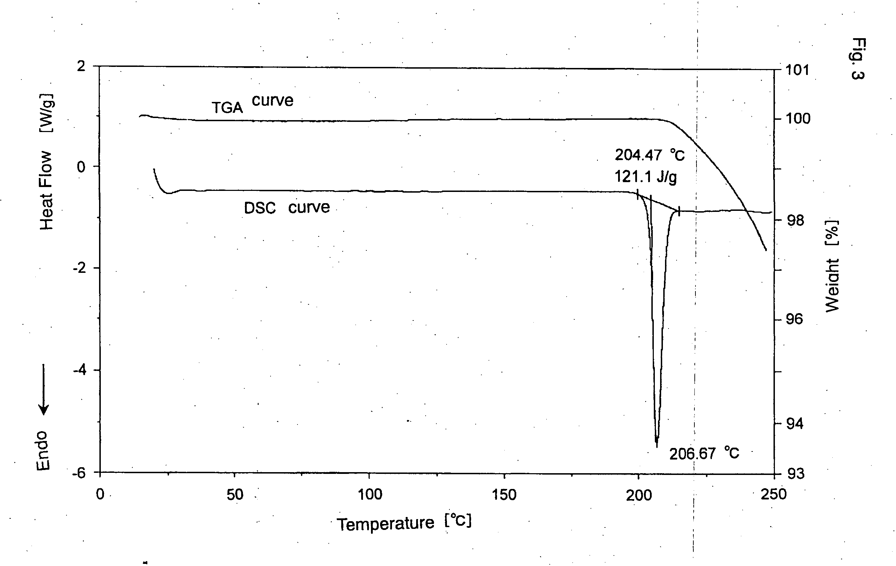

A chart showing the X-ray powder diffraction data of the compound of Example 3 is shown in Fig. 1, a chart showing a TGA-DSC thermal analysis data is shown in Fig. 3, a chart showing a hygroscopicity is shown in Fig. 5 and elementary analysis data are shown in Table 3.

**(Table 3)**

| Elementary Analysis of Compound of Example 3 | | | | | | |
|---|---|---|---|---|---|---|
| | C (%) | H (%) | N (%) | O (%) | F (%) | P (%) |
| Calculated | 55.02 | 6.22 | 7.40 | 22.55 | 3.35 | 5.46 |
| Found | 55.01 | 6.15 | 7.43 | | 3.29 | 5.44 |

### Example 4

### (Manufacture of α-type crystal of compound A)

To 80.0 g of the compound of Example 3 were added 800 ml of EtOH and 91 ml of water followed by heating to dissolve and the solution was filtered. The filtrate was heated with stirring and cooled, a compound having the same crystal form as in Example 1 was added thereto as a seed crystal at about 60°C and the mixture was cooled down to about 20°C and crystallized with stirring. The crystals separated out therefrom were filtered, washed with EtOH and dried *in vacuo* to give 67.08 g of (-)-N-{2-[(R)-3-(6,7-dimethoxy-1,2,3,4-tetrahydroisoquinoline-2-carbonyl)piperidino]ethyl}-4- fluorobenzamide monophosphate (α-type crystal) having a purity of 99.7%.

### Industrial Applicability

In accordance with the present invention, there are provided α-type crystal and β-type crystal of a compound A having an inhibitory action for I_{f} current and showing a strong and specific activity selectively lowering a heart beat and decreasing oxygen consumption of heart muscle in a selective manner whereby being useful as a preventive and/or treating agent for diseases of circulatory system such as ischemic heart diseases (e.g., angina pectoris and myocardial infarction), congestive heart failure, arrhythmia, etc. The crystal as such is α-type crystal or β-type crystal of the pure compound A or a mixture which is able to be considered to be identical with pure α-type crystal and β-type crystal as such. Accordingly, in accordance with the present invention, it is now possible to always provide in a constant manner a single crystal being able to ensure the uniform quality and certain function and effect which are requested for pharmaceuticals.

### Brief Description of Drawings

Fig. 1 is a chart which shows the X-ray powder diffraction data of α-type crystal of the compound A (Example 3) in which an ordinate shows diffraction intensity while an abscissa shows diffraction angle (2θ).
Fig. 2 is a chart which shows the X-ray powder diffraction data of β-type crystal of the compound A (Example 2) in which an ordinate shows diffraction intensity while an abscissa shows diffraction angle (2θ).
Fig. 3 is a chart which shows the TGA-DSC thermal analysis data of α-type crystal of the compound A (Example 3).

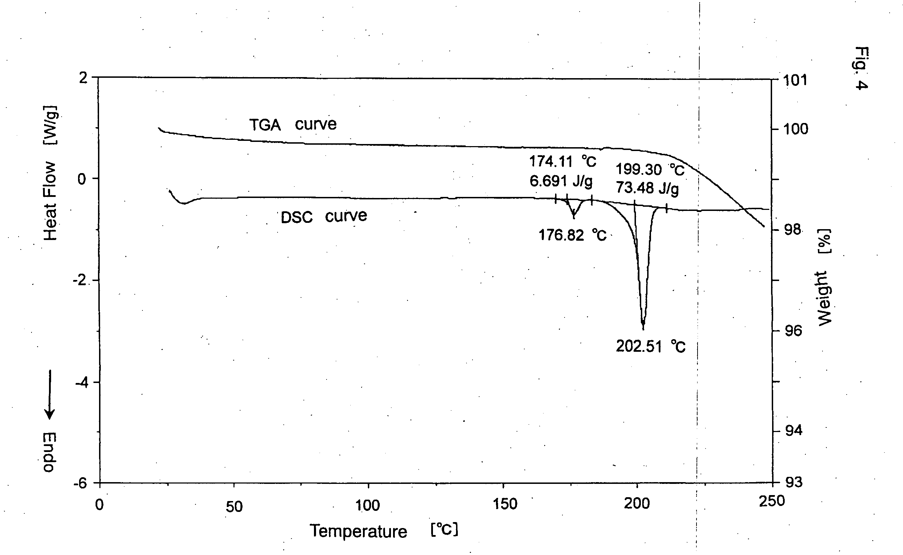

Fig. 4 is a chart which shows the TGA-DSC thermal analysis data of β-type crystal of the compound A (Example 2).
Fig. 5 is a chart which shows the hygroscopicity of α-type crystal of the compound A (Example 3).
Fig. 6 is a chart which shows the hygroscopicity of β-type crystal of the compound A (Example 2).

## Claims

1. Crystal of (-)-N- {2-[(R)-3-(6,7-dimethoxy-1,2,3,4-tetrahydroisoquinoline-2-carbonyl)piperidino]ethyl}-4-fluorobenzamide monophosphate which is **characterized in** having main peaks near 19.36, 6.23, 4.73 and 3.81 Å of lattice spacing in a X-ray powder diffraction pattern obtained by the use of Cu-Kα radiation.

2. The crystal according to claim 1, wherein lattice spacing and relative intensity in a X-ray powder diffraction pattern obtained by the use of Cu-Kα radiation are as shown in Table 4.
**(Table 4)**
| Lattice Spacing (Å) | Relative Intensity |
|---|---|
| 19.36 | strong |
| 15.28 | slightly strong |
| 6.23 | strong |
| 5.71 | slightly strong |
| 4.73 | strong |
| 4.50 | slightly strong |
| 3.81 | strong |

3. Crystal of (-)-N- {2-[(R)-3-(6,7-dimethoxy-1,2,3,4-tetrahydroisoquinoline-2-carbonyl)piperidino]ethyl}-4-fluorobenzamide monophosphate which is **characterized** that, in a DSC curve, the temperature of endothermic peak is near 206 to 207°C.

4. Crystal of (-)-N- {2-[(R)-3-(6,7-dimethoxy-1,2,3,4-tetrahydroisoquinoline-2-carbonyl)piperidino]ethyl}-4-fluorobenzamide monophosphate which is **characterized in** having main peaks near 18.40, 6.52, 5.22, 4.38 and 4.09 Å of lattice spacing in a X-ray powder diffraction pattern obtained by the use of Cu-Kα radiation.

5. The crystal according to claim 4, wherein lattice spacing and relative intensity in a X-ray powder diffraction pattern obtained by the use of Cu-Kα radiation are as shown in Table 5.
**(Table 5)**
| Lattice Spacing (Å) | Relative Intensity |
|---|---|
| 18.40 | strong |
| 6.52 | strong |
| 5.22 | strong |
| 5.06 | slightly strong |
| 4.86 | slightly strong |
| 4.38 | strong |
| 4.09 | strong |
| 3.74 | slightly strong |

6. Crystal of (-)-N- {2-[(R)-3-(6,7-dimethoxy-1,2,3,4-tetrahydroisoquinoline-2-carbonyl)piperidino]ethyll-4-fluorobenzamide monophosphate which is **characterized** that, in a DSC curve, the temperature of endothermic peak is near 176 to 177°C.
